(19) Europäisches Patentamt
European Patent Office
Office européen des brevets

(11) **EP 4 124 302 A1**

(12) **EUROPEAN PATENT APPLICATION**

(43) Date of publication:
**01.02.2023 Bulletin 2023/05**

(21) Application number: **21315133.5**

(22) Date of filing: **29.07.2021**

(51) International Patent Classification (IPC):
**A61B 8/08** (2006.01)        **G01S 7/52** (2006.01)

(52) Cooperative Patent Classification (CPC):
**A61B 8/485; G01S 7/52042; A61B 8/085;**
A61B 8/429; A61B 8/483; A61B 8/5215;
A61B 8/5253

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **SuperSonic Imagine
13857 Aix-en-Provence Cedex 3 (FR)**

(72) Inventors:
• **Etaix, Nicolas
13100 Aix-en-Provence (FR)**
• **Zhang, Bo
13109 Simiane-Collongue (FR)**

(74) Representative: **Paustian & Partner Patentanwälte
mbB
Oberanger 32
80331 München (DE)**

(54) **ULTRASONIC METHOD AND SYSTEM FOR ESTIMATING THE NONLINEAR SHEAR WAVE ELASTICITY OF A MEDIUM**

(57)    The invention relates to an ultrasonic method for estimating a nonlinear shear wave elasticity of a medium, the method comprising the following steps:
• A1. a first collection step in which a first set comprising one shear wave elasticity data point of the medium is collected at a first level of deformation applied to the medium,
• A2. a second collection step in which a second set comprising one shear wave elasticity data point of the medium is collected at a second level of deformation applied to the medium different to the first level,
• A3. a deformation estimation step in which the difference of deformation between the first and the second level of deformation is estimated,
• B1. a calculation step in which a gradient between at least two data points respectively belonging to the first and the second set is calculated as a function of the difference of deformation between the first and the second level of deformation,
• B2. an elasticity estimation step in which the nonlinear shear wave elasticity of the medium is estimated as a function of the gradient.

Fig. 1

**Description**

FIELD OF THE DISCLOSURE

The present disclosure relates to ultrasound technologies and more particularly imaging methods and systems for providing image data of a viscoelastic medium. In particular, the method is intended to estimate the non-linear shear wave elasticity (NL-SWE) of the medium, more in particular to detect a soft cancerous region (for example in the breast or, liver) in the medium (i.e., cancerous compared to non-cancerous medium).

BACKGROUND OF THE DISCLOSURE

Ultrasound data used for building images can be obtained by different processes. For example, the obtained images may be static deformation (i.e. strain) images, obtained by deformation of the observed tissue, or shear wave elastography (SWE) images.

It is known to detect indurated cancers by shear wave elastography (SWE) ultrasound imaging. However, this technique may be less suitable for detecting soft cancers, mainly because soft cancers may have similar elasticity characteristics compared to non-cancerous media on a conventional elastography image.

It is further known to estimate non-linearity characteristics of a medium, for example from H. Latorre-Ossa et.al: Quantitative imaging of nonlinear shear modulus by combining static elastography and shear wave elastography, IEEE Transactions on Ultrasonic Ferroelectronics and Frequency Control. 2012 Apr; 59(4):833-9. This work proposes a method combining static elastography and shear wave elastography to derive the nonlinearity shear modulus by applying the acousto-elasticity theory in quasi-incompressible soft solids. A mechanical actuator is used to axially displace a probe and a compressor plate attached to it. The quasi-static stress is thereby applied at the top of a phantom by axially displacing the probe and the compressor plate in steps of 0.1 mm (~0.33% with each step lasting less than 30s). At each compression step, a complete shear velocity map is retrieved and, therefore, the 2-D shear modulus map of the medium.

A further work is known from M. Bernal et.al.: In Vivo Quantification of the Nonlinear Shear Modulus in Breast Lesions: Feasibility Study. IEEE Trans Ultrasonic Ferroelectric Frequency Control. 2016 Jan; 63(1): 101-9. doi: 10.1109/TUFFC.2015.2503601. Epub 2015 Nov 24. In this work, phantoms are compressed uniaxially using a linear motor. The motor has programming steps and therefore provides a controlled uniaxial stress to the phantom. Immediately after a given step compression, shear wave velocity maps are obtained. From these data, shear modulus maps or Young's modulus maps are also retrieved.

However, the concept of employing mechanically controlled deformation devices does not match with a practical application in the field of medical examination. In this practice, a user (for example a doctor, a technician, a sonographer) needs to manually control a hand-held ultrasound probe, thereby taking into account a patient's individual situation and body (for example whether the examination is painful or at least uncomfortable for the patient). As a consequence, it would be impossible or at least very difficult for the user to achieve a regularized gesture, for example a regularized stepwise deformation. Instead, the manual compression leads to several inaccuracies, for example regarding the required deformation of the medium or any potential variations of deformation within a compression step. Such inaccuracies can lead to noisy data and hence to an inaccurate estimation of non-linear shear wave elasticity (NL-SWE) value(s). For example, the way of applying the stress can differ from one practitioner to another. Such a user-dependency requires a different method than the above-mentioned ones, in order to improve the acquisition repeatability.

As a possible counter measure the number of compression steps might be limited. However, in this case also the total amount of collected data in all steps is reduced, respectively. Hence, even if the user might more accurately apply less compression steps, any remaining noise in the data would have an even stronger influence on the final result.

A further ultrasonic method for quantifying the non-linear elasticity of a medium using shear waves is known from WO2021116326A2. The method comprises the following steps: A1. - collecting a temporal succession of data on the elasticity of the medium using shear waves, A2. - applying, to the medium, a deformation that successively changes according to a predetermined sequence of deformations, during the collection of the shear waves, A3. - observing the actual deformation variation, and B. - quantifying the non-linear elasticity of the medium depending on the temporal succession of data and the deformation variation. The method allows a continuously and progressively applied deformation of the medium.

However, the amount of collected data obtained by the method can still be limited due to temperature and duration constraints. An ultrasound probe used for collecting data and applying the deformation may namely heat the (human tissue) medium. Moreover, an examined human has to stay still in·a given, maybe unnatural position. For these reasons, the data collection should take as less time as possible. Furthermore, the data quality can be limited, for example due to user fluctuations and noisy measurements.

Summarized, the known methods show limited robustness against small data and high noise, and a limited reproducibility in practical use, leading in particular to an increased complexity to analyze the results. The analysis may thus require more time and effort and may potentially require several exams of the patient to collect enough meaningful information.

SUMMARY OF THE DISCLOSURE

Currently, it remains desirable to overcome the aforementioned problems and in particular to provide an ultrasonic method and system for accurately estimating the nonlinear shear wave elasticity of a medium, more in particular, in case there is only a limited amount of collected data. In other words, the desired method should be more robust against small data and high noise and thus have a better reproducibility.

Accordingly, the purpose of the present disclosure is hence to obtain complementary and more meaningful information of a region of interest in a medium which may facilitate analysis of the medium in a practical application. It is consequently desirable to reduce the time for analysing the acquired examination information and to shorten examination time of a patient as far as possible. Non-limiting examples of a medical application comprise a detection of non-iridurated cancer, in particular in a non-invasive manner (avoiding for example any intervention such as biopsy to obtain information on the region of interest).

Therefore, according to the embodiments of the present disclosure, a method for estimating a nonlinear shear wave elasticity of a medium. Said method comprises the following steps:

- A1. a first collection step in which a first set comprising one shear wave elasticity data point of the medium is collected at a first level of deformation applied to the medium,
- A2. a second collection step in which a second set comprising one shear wave elasticity data point of the medium is collected at a second level of deformation applied to the medium different to the first level,
- A3. a deformation estimation step in which the difference of deformation between the first and the second level of deformation is estimated,
- B1. a calculation step in which a gradient between the data points of at least two data points respectively belonging to the first and the second set is calculated as a function of the difference of deformation between the first and the second level of deformation,
- B2. an elasticity estimation step in which the nonlinear shear wave elasticity of the medium is estimated as a function of the gradient.

By providing such a method, it becomes possible to obtain a method for estimating a nonlinear shear wave elasticity which is more robust against a small amount of collected data and high noise in the data. The method thus allows a more reliable estimation and a better reproducibility, even if the change of deformation levels is made manually.

Accordingly, the proposed method is user-independent predictable and does not require detailed training, thereby improving the acquisition repeatability. In other words, the circumstance that the way of applying the stress (i.e. deformation of the medium) may differ from one practitioner to another, is not influencing or at least less influencing the quality of the acquired examination information.

As a further advantage, the required time and associated costs for examining a patient can be reduced. This is in particular advantageous, avoiding the probe to become warm due to ultrasound waves and because an ultrasound examination can be tiring or uncomfortable for a patient due to an uncomfortable position during examination. Moreover, also the required time, costs and complexity of analyzing the acquired information can be reduced due to its increased meaningfulness.

Advantageously the method of the present disclosure may be carried out with a conventional ultrasound system which is configured for SWE imaging. In other words, no hardware modifications are necessary.

The gradient may be determined by a variation of shear wave elasticity between the data points as a function of the difference of deformation between 'the first and the at least one second level of deformation.

In one example, the gradient $R_{ij}$ may be determined by:

$$ R_{ij} := \frac{E_i - E_j}{\epsilon_i - \epsilon_j} $$

where E is a Young's modulus coefficient of the medium determined as a function of the wave elasticity data points and $\epsilon$ is a strain coefficient representing the level of deformation, wherein indices i and j represent two different levels of deformation.

In another example, the gradient $R_{i_1,...,i_k}$ may be determined by (i.e. generalized to):

$$R_{i_1,i_2,...,i_k} := \frac{k \sum_{s=1,2,...,k} \epsilon_{i_s} E_{i_s} - \sum_{s=1,2,...,k} \epsilon_{i_s} \sum_{s=1,2,...,k} E_{i_s}}{k \sum_{s=1,2,...,k} \epsilon_{i_s}^2 - \left(\sum_{s=1,2,...,k} \epsilon_{i_s}\right)^2}$$

where $i_1, i_2, ..., i_k$ are indices representing $k$ ($k \geq 2$) different levels of deformation. Accordingly, the slope defined on pairwise points (cf. for example eq. (2) above) may thus be generalized to slopes defined on a subset of points of size k (with k>2).

The deformation estimation step may comprise the sub-steps of:

- collecting a succession of strain data (or strain data points) of the medium at the different levels of deformation,
- determining the difference of deformation between the first and the second level of deformation as a function of the strain data.

The sub-step of collecting a succession of strain data may comprise:

- generating a succession of ultrasound data of the medium, and
- comparing the ultrasound data using a predefined deformation estimation algorithm.

Said succession of ultrasound data may comprise for example B-mode ultrasound data. The succession may be a temporal succession, for example comprising a plurality of consecutive image frames data.

In one example, the strain data points and SWE data points may be collected substantially at the same time. However, the data points may also be collected alternately, for example using a single ultrasound transducer or transducer network.

Said algorithm may for example be an AI-based algorithm (artificial intelligence). The algorithm may for instance comprise a computer-implemented machine learning model, such as an artificial neural network. Examples comprise a Convolutional neural network. The model may be trained by a supervised training method using for example annotated images as training data, or by an unsupervised training method.

Said algorithm may be based on the determination of at least one of:

- a pixel correlation between successive frames,
- an auto-correlation phase between successive frames,
- a doppler signal between successive frames,
- speckle tracking between successive frames, and
- an optical flux between successive frames,
- or any other data comparison technique.

In one example, the strain data may be collected using a 2D probe (i.e. a probe configured to acquired two-dimensional image data). In another example, the strain data may be collected using a 3D probe (i.e. a probe configured to acquired three-dimensional image data). In this case three-dimensional strain data may be obtained, what can lead to a more precise deformation estimation. Moreover, it may then be determined based on the strain data whether the probe moves transversally along an axis or in an undesired rotational direction.

A shear wave elasticity data point may comprise a plurality of shearwave values referring respectively to different regions (or areas) of a ROI (region of interest) of the medium. For each region (area) a nonlinear shear wave elasticity parameter is estimated for constructing a nonlinear shear wave elasticity map of the region of interest.

Moreover, it is accordingly possible that the succession of strain data (or strain data points) may comprise a plurality of strain values referring respectively to the different regions (or areas) of the ROI (region of interest) of the medium. However, it is also possible that the succession of strain data (or strain data points) may comprise only one strain value (i.e. in a temporal succession) representing the ROI (region of interest) of the medium.

For example, a shear wave elasticity data point may comprise a shear wave elasticity image data with a plurality of pixels. For example, a SWE data point may constitute an SWE image frame in a series of respective frames.

For each pixel a nonlinear SWE parameter may be estimated for constructing a nonlinear shear wave elasticity map. Accordingly, said NL-SWE map may have the same pixel resolution like the SWE image data. It is though also possible that the NL-SWE map has a reduced resolution, for example by estimating a single NL-SWE parameter for a pixel cluster.

At least one of the first and second set may comprise a plurality of shear wave elasticity data points. For example, each set comprises a plurality of SWE data points.

In the calculation step, a plurality of gradients between at least two data points, respectively belonging to the first and the second set may be calculated as a function of the difference of deformation between the first and the second level of deformation.

In the elasticity estimation step, the nonlinear shear wave elasticity of the medium may be estimated as a function of the plurality of gradients.

Accordingly, given N SWE data points, the data quantity of calculated gradients when any two different levels are concerned can be up to (N - 1)$N/2$ (cf. eq. (2)). Hence, in the example of eq. (2) the data size to deal with can be increased from $N$ to $(N - 1)N/2$ without increasing the data collection duration. Moreover, when all subsets of $k(k \geq 2)$ points among the N points are concerned, the size of the calculated gradients increases to ($2^N - N - 1$) (cf. eq. (4)). Hence, in the example of eq. (4) the data size to deal with can be even increased from $N$ to ($2^N - N - 1$) without increasing the data collection duration.

Desirably the collected SWE data points are substantially equally distributed over the levels of deformation. In other words, each level of deformation may comprise substantially the same number of collected SWE data points. In case the SWE data points are collected with a predefined sampling rate, the equal distribution may for example be obtained by guiding the user (for example using a user interface) to stay within each level of deformation substantially for the same time. The advantage of such an equal distribution is that the number of possible combinations of data point of different levels of deformations can be increased. Accordingly, the total data size (i.e. the number of gradients) to deal with in the NL-SWE estimation can be increased.

The elasticity estimation step may further comprise the sub-steps of:

- building a linear minimum-variance estimator based on the estimated gradients ($R_{ij}$ and/or $R_{i_1, i_2, ..., i_k}$) and their statistical variances for estimating a score ($w_{ij}$ and/or $w_{i_1, i_2, ..., i_k}$) for each gradient which is inversely proportional to its variance. the statistical variances may be for example spatial and/or temporal statistical variances,
- weighting each gradient with the respective score,
- estimating a final NL-SWE parameter as a function of the weighted gradients and/or a weighted average of the gradients.

Accordingly, an estimator of estimating the NL-SWE parameter may be used that has minimum variance among all linear estimators. Said NL-SWE estimator is thus more robust than an ordinary least squares linear fit.

For example, the linear minimum-variance estimation of the NL-SWE $\hat{A}$ may be determined by

$$\hat{A} = \sum_{ij} w_{ij}^* A_{ij}$$ with the optimal weighting scores are derived from the following optimization:

$$\arg \min_{w^*} Var\left(\sum_{ij} w_{ij} A_{ij}\right) \quad s.t. (subject\ to) \ \sum_{ij} w_{ij} = 1$$

where:

Var designates the variance,
$w_{ij}$ is a weighting score to be derived,
$A_{ij}$ is a nonlinear shear wave elasticity parameter derived from the gradient Rij, and
indices i and j represent two different levels of deformation.

Moreover, a Geary-Hinkley Transformation may be used for improving the accuracy of the minimum variance estimator.

The optimal score $w_{ij}^*$ may be estimated by:

$$w_{ij}^* = \frac{\sigma_{A_{ij}}^{-2}}{\sum_{mn} \sigma_{A_{mn}}^{-2}}$$

, where

$w_{ij}^*$ is the score derived from the variances of each nonlinear shear wave elasticity parameter $A_{ij}$,

$\sigma_{A_{ij}}^{-2}$ is an inverse variance estimate of the nonlinear shear wave elasticity parameter $A_{ij}$, and

$\sigma_{A_{mn}}^{-2}$ is an inverse variance estimate of the nonlinear shear wave elasticity parameter $A_{mn}$, wherein indices m and n run through at least two (or up to all) pairwise combinations of shear wave elasticity data points by: $1 <= m <= N$, $m < n <= N$, where N is the number of data points considered in the method for estimating the NL-SWE. N may be desirably at least 2, more desirably at least 3. In this context it is noted that the method works with only 2 SWE data points. However, with N being 3 or more, the data size to deal with in the NL-SWE estimation step can be increased from *N* to *N(N-1)/2* without increasing the acquisition duration.

As already mentioned, the final NL-SWE parameter $\hat{A}$ may be estimated by:

$$\hat{A} = \sum_{ij} w_{ij}^* A_{ij}$$

The method may further comprise a confidence estimation step comprising:

• determining a statistical model on the confidence of the minimum-variance estimator as a function of the at least one score and the variance estimate $(\sigma_{A_{ij}}^2)$ of the nonlinear shear wave elasticity parameter ($A_{ij}$),
• generating a confidence map comprising confidence information for the regions or pixels of the nonlinear shear wave elasticity map.

Accordingly, said confidence map may indicate, in which areas the NL-SWE map appears more reliable and in which less.
Said confidence map may have the same pixel resolution like the NL-SWE map. It is though also possible that the confidence map has a reduced resolution, for example by estimating a single confidence parameter for a pixel cluster of the NL-SWE map.
A level of deformation may be defined by a range of deformation, such that only any shear wave elasticity data point within the range of a given level is collected and/or considered in the calculation step.
The ranges of two adjacent levels of deformation desirably do not overlap. Accordingly, a sufficient deformation change between two levels of deformation can be achieved.
During a change from one level of deformation to another only strain data may be collected. In other words, SWE data points during such a change are not collected or disregarded in the and/or the NL-SWE estimation method.
The same ultrasound probe may be used to emit and receive ultrasound waves, to collect the shear wave elasticity data points and/or to collect the strain data and/or to apply the deformation to the medium. Accordingly, a user of the probe may collect the required date from the medium and at the same time apply manually the deformation using said probe to the medium.
The method may further comprise a guidance and/or feedback step in which a user is guided by a user interface to apply the deformation to the medium and/or receives feedback from the user interface as a function of the applied deformation.
Accordingly, a user may apply the deformation freely and manually but may be assisted by the method, for example to respect a required minimum time for data collection in each level of deformation and/or a required minimum deformation change between two levels of deformation.
The present disclosure further relates to a computer program comprising computer-readable instructions which, when executed by a data processing system, cause the data processing system to carry out the method according to the present disclosure.
The present disclosure may also relate to a recording medium readable by a computer and having recorded thereon a computer program including instructions for executing the steps of the method according to the present disclosure, when said program is executed by a computer.
The present disclosure further relates to an ultrasonic system for estimating a nonlinear shear wave elasticity of a medium, the system being ' configured to:

- collect a first set comprising one shear wave elasticity data point of the medium at a first level of deformation applied to the medium,
- collect a second set comprising one shear wave elasticity data point of the medium at a second level of deformation applied to the medium different to the first level,
- estimate the difference of deformation between the first and the second level of deformation,
- calculate a gradient between the data points of at least two data points respectively belonging to the first and the second set as a function of the difference of deformation between the first and the second level of deformation,

estimate the nonlinear shear wave elasticity of the medium is estimated as a function of the gradient.

The system may comprise further functional characteristics and/or may be configured corresponding to the method steps described above.

The disclosure and its embodiments may be used in the context of medical systems dedicated to human beings, plants or animals but also any (potentially non-living) soft material to be considered.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, are provided for illustration purposes, and illustrate embodiments of the disclosure and together with the description and serve to support and illustrate the principles thereof.

BRIEF DESCRIPTION OF THE DRAWINGS

Fig. 1 shows a first exemplary embodiment of the method according to embodiments of the present disclosure;

Fig. 2A, 2B and 2C schematically show an example of a stepwise deformation of a medium according to embodiments of the present disclosure;

Fig. 3a schematically shows a diagram of strain as a function of time frames according to embodiments of the present disclosure;

Fig. 3b schematically shows a diagram of elasticity E as a function of time frames corresponding to the strain function of fig. 3a;

Fig. 4 shows an exemplary flow chart of the method according to embodiments of the present disclosure in more detail;

Fig. 5 shows an exemplary embodiment of an ultrasound system according to embodiments of the present disclosure.

DESCRIPTION OF THE EMBODIMENTS

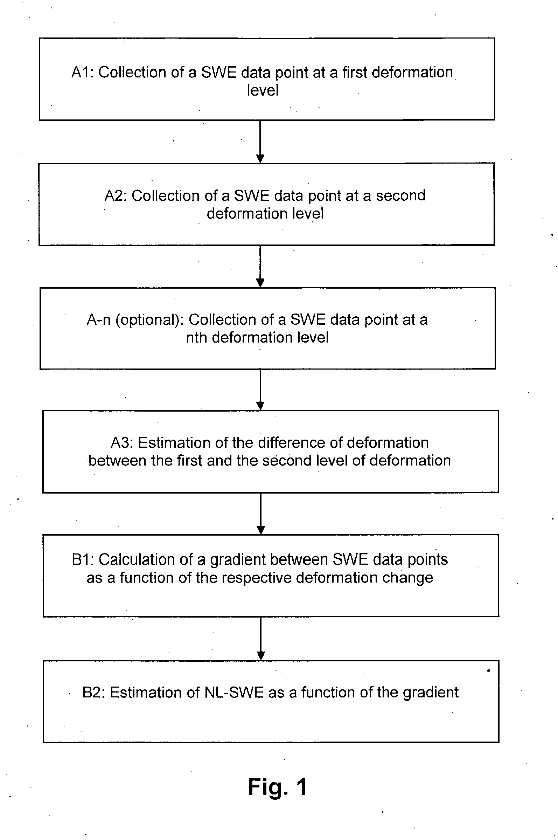

Reference will now be made in detail to exemplary embodiments of the disclosure, examples of which are illustrated in the accompanying drawings. Wherever possible, the same reference numbers will be used throughout the drawings to refer to the same or like parts. Moreover, the features explained in context of a specific embodiment, for example that one of fig. 1, also apply to any one of the other embodiments, when appropriate, unless differently described.

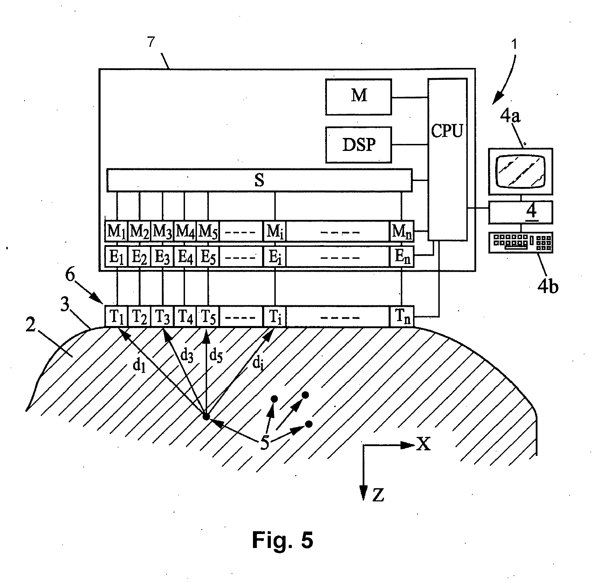

Fig. 1 shows a first exemplary embodiment of the method according to embodiments of the present disclosure. The method may be carried out by means of an ultrasound system. An example is described in context of fig. 5. For example, to begin/start this process, a first imaging mode for nonlinear shear wave elasticity (NL-SWE) may be activated. The method comprises the following steps:

In a step A1 a first set comprising at least one SWE (shear wave elasticity) data point of the medium is collected at a first level of deformation applied to the medium. Exemplary embodiments of a collection method and of an SWE data point are described below. The first level of deformation may also be considered as a reference level. Said first level may also be at a level of no or almost no deformation applied to the region of interest, i.e. where for example the probe is in contact (using gel) with the medium without any applied stress..

In a step A2 a second set comprising at least one shear wave elasticity data point of the medium is collected at a second level of deformation applied to the medium. Said second level is different to the first level, for example more deformed or less deformed than the first level. The second level of deformation may also be considered as a first comparative level. In case the first level implies a deformation of the medium, the second level may also be at a level of no or almost no deformation applied to the region of interest, i.e. where for example the probe is in contact (using gel) with the medium without any applied stress.

In an optional step A-n an $n^{th}$ set comprising at least one shear wave elasticity data point of the medium is collected at an $n^{th}$ level of deformation applied to the medium. There may exist also a plurality of $n^{th}$ levels of deformation, for example a third level, a fourth level and so on. Said nth level may be different to the second level and/or the first level. For example, the first, second and $n^{th}$ levels may form a successively changing deformation, for example in a continuously increasing or decreasing deformation. Alternatively, they may also be alternating levels of deformation. For example, the $n^{th}$ level of deformation may substantially correspond to the first level of deformation.

In a step A3 the difference of deformation between the first and the second level of deformation is estimated. Optionally also the difference between the $n^{th}$ level and the second level and/or the first level is estimated. An exemplary estimation method is described in the following.

In a step B1 at least one gradient (i.e. a ratio or variation) between at least two data points respectively belonging to the first, the second set and/or the $n^{th}$ set is determined or optionally belong to at least three data points respectively belonging to the first, the second set and the $n^{th}$ set is determined. Said gradient may in particular be determined by a variation of shear wave elasticity between the data points as a function of the difference of deformation between the respective first, the second and optionally the $n^{th}$ level of deformation. For example, a first gradient may be determined as a function of two data points of the first and the second set and an optional second gradient may be determined as a function of two data points of the second and the $n^{th}$ set. A further exemplary gradient may be determined as a function of two data points of the first and the $n^{th}$ set, in particular, in case the respective first and $n^{th}$ level of deformation are different. Moreover, a gradient may also be determined as a function of more than two data points.

In a step B2, the NL-SWE (nonlinear shear wave elasticity) of the medium (for example a NL-SWE parameter) is estimated as a function of the at least one gradient.

Steps A1 to A-n may be successive in time. Step A3 may desirably be carried out at the same time as steps A1, A2, A-n. Advantageously, this process (i.e. the respective SWE data collection or "scan") may be combined simultaneously and/or interleaved with an ultrasonic process to estimate the evolution of the deformation (i.e. step A3) in real time or near real time.

Step B1 may be carried out once after step A3, and/or several times starting after step A2 and being repeated after each further step A-n. In this case the respective gradients of the already collected data points may be determined in the respective steps B1. Step B2 may be carried out after step B1 or several times after respective several steps B1. In the latter case, a first estimation may be obtained rapidly in a first step B2 which may then be further refined in subsequent steps B2. The method A1 to B2 may also be repeated by a respective loop from B2 back to A1.

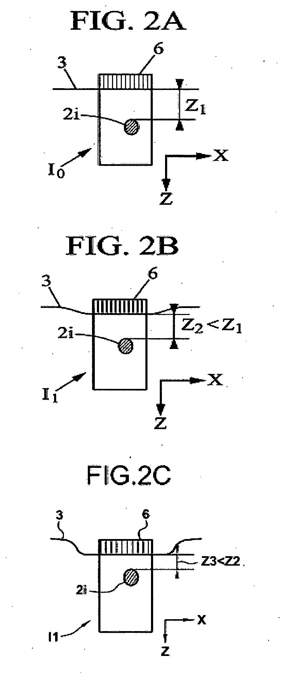

Optional embodiments and aspects of the method are described in the following, also in context of figures 2 to 5.

A shear wave elasticity data point may comprise a shear wave elasticity image with a plurality of pixels, wherein for each pixel a nonlinear shear wave elasticity parameter is estimated for constructing a nonlinear shear wave elasticity map. In other words, a SWE data point may be understood as one data collection cycle from which a SWE image of a region of interest of the medium may be derived.

According to one aspect, a shear wave elasticity data point produced in step (A1) may be generated by the following sub-steps:

A1.1. an excitation step in which a shear wave is generated in the medium by causing at least one focused ultrasonic wave to be emitted,

A1.2. an observation step during which the propagation of the shear wave is observed by acquiring a temporal succession of ultrasound data from the medium,

A1.3. a processing step during which the data of the elasticity data point are determined from said ultrasonic data of the medium and from a shear wave propagation model.

This process may use plane waves, but this step may also be performed by using focused waves.

Accordingly, the NL-SWE method may use an ultrasonic method to determine the shear wave elasticity (SWE) of the medium.

Corresponding sub-steps may be comprised by steps A2 and A-n.

The deformation estimation step A3 may comprise the sub-steps of collecting a succession of physical strain data of the medium at the different levels of deformation and determining the difference of deformation between the first and the second level of deformation as a function of the strain data. The sub-step of collecting a succession of physical strain data may in particular comprise generating a succession of ultrasound image frames of the medium and comparing the ultrasound image frames using a predefined movement estimation algorithm.

Also the change of deformation between the first and the $n^{th}$ and/or the second and the $n^{th}$ level of deformation may be determined by corresponding sub-steps.

For example, the ultrasound probe which is used to collect the shear wave elasticity data points may also be used to collect the strain data and/or to apply the deformation to the medium.

The shearwave data collection and deformation estimation methods may be based on planar ultrasound waves, while a B-mode method may be based on non-planar ultrasound waves. As an alternative to the ultrasonic process for determining the static elasticity ("strain") representing the change of deformation, or in addition, the probe may also be equipped with a pressure transducer that allows to observe (measure or follow) the deformation evolution of the medium.

Figures 2A, 2B and 2C schematically show an example of a deformation of a medium according to embodiments of the present disclosure. In particular, fig. 2A to 2C schematically show an application of a successively changing (i.e. increasing) deformation to the medium. However, also a decreasing or an alternating change of deformation may be applied. In the example of fig. 2A to 2C, the process begins in the state illustrated in Fig. 2A and ends in the state illustrated in Fig. 2C. In particular, fig. 2A to 2C show the deformation evolution of the medium, caused by an application of a for example stepwise increasing deformation.

Each of fig. 2A to 2C may hence show one step, i.e. one level of deformation. For example, fig. 2A may represent a first level of deformation according to the disclosure, fig. 2B may represent a second level of deformation according to the disclosure, and fig. 2C may represent a $n^{th}$ level of deformation according to the disclosure. Advantageously the method of the present disclosure may allow the user to freely select or choose the levels of deformation. In other words, the levels are desirably not predefined. This is possible because the method advantageously collects all necessary data to reliably calculate the gradient(s), independently of the change(s) of deformation(s) applied by the user.

For example, a user may hold the probe 6 in a first position in relation of the medium, as for instance shown in fig. 2A for a certain time (for example from a fraction of second to several seconds or as indicated to the user by a user interface) in a substantially constant position, then changes the position to the second level of deformation as shown for instance in fig 2B to keep constant again for a certain period of time, and so on. In each substantially constant position at least one SWE data point may be collected, and the level of deformation may be estimated accordingly, in real time, pseudo real time or post processing. Accordingly, in the latter case of a post processing it is also possible that the examined person may leave, after the data have been collected (cf. steps A1, A2 of the method), and before the data are processed to estimate the NL-SWE. It is further possible that the data processing is done remotely, for example in another room or building than the one where the data collection took place. A level of deformation may hence be defined by a (predefined tolerance) range of deformation, such that only shear wave elasticity data points within the range of a given level is collected and/or may be considered in the calculation step. Once the user applies a change of deformation exceeding the (tolerance) range, the method may recognize a change of deformation from one level to another. The ranges of two adjacent levels of deformation (for example of fig. 2A and 2B) are desirably chosen such they do not overlap. In this way the method can reliably recognize whether the deformation has been changed from one level to another. During a change from one level of deformation to another only strain data may be collected/registered/sorted.

Fig. 2A depicts an operation of the ultrasound system 1 at a time t0 of the process, with the probe 6 exerting less pressure P on the external surface 3 than in the subsequent steps. The external surface 3 remains substantially horizontal (in the X direction). The image data of the medium I0 includes, for example, an inclusion 2i at a depth Z1 relative to the external surface 3. At the same time, the ultrasonic process for estimating the nonlinear shear wave elasticity of the medium (as explained above) starts.

Fig. 2B shows an operation of the ultrasound system 1 at a time t1 of the process, and with an external pressure (or stress) exerted, greater than P, which deforms the outer surface 3 in the Z direction towards the interior of the medium 2. Thus, the deformation of the medium has been successively changed.

Fig. 2C shows an operation of the ultrasound system 1 at a time t2 of the process, with an external pressure P" even higher than P' exerted at t1. The changing deformation, as illustrated in fig. 2A to 2C, may be carried out in a stepwise manner. Alternatively, a successive decompression or an alternating compression/decompression may be applied to the medium.

The complete process may take a few seconds, for example between 5-10s. Each step may take for example 0,5-5s. Desirably, the method does not imply any upper time limit. However, due to external time constraints of for example the patrician and /or examined person the method and in particular the data collection (steps A1, A2 of the method) is carried out rapidly. The examined person may for example be required to stay still in an uncomfortable or tiring position or the examined part of the body may be heated may ultrasound waves. The user may freely control the time of each single deformation level but may be guided by a user interface (as described below). This time is useful to collect enough data during the deformation evolution of the medium (i.e., to sufficiently quantify the nonlinear shear wave elasticity of a medium during this evolution).

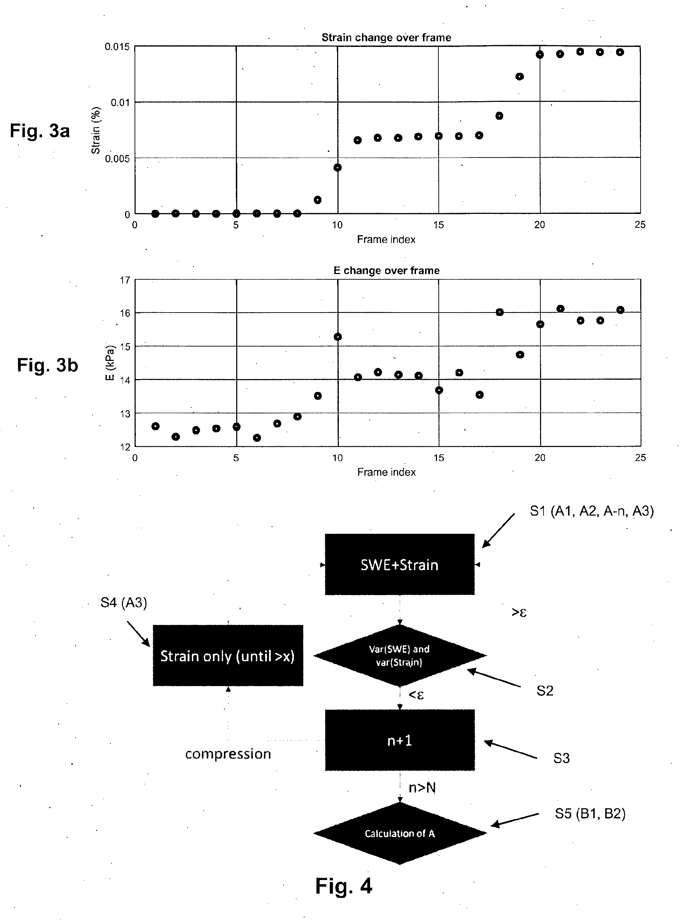

In other words, the deformation evolution shown in fig. 2A to 2C may be freely chosen by the user but may also be guided by a predefined deformation sequence or an adaptative deformation sequence (i.e.; built in real time and/or on a case-by-case basis by an computer algorithm, for example an AI based algorithm,). For example, this sequence may be indicated by a user interface comprising at least one of a visual indicator, an acoustic indicator and a haptic indicator. Examples of a visual indicator include an arrow or any other symbol on a screen or displayed in another way, and LEDs, for example placed on the ultrasound probe. Examples of an acoustic indicator include voice guidance or alarm sounds indications. Examples of a haptic indicator include a vibration unit, incorporated for example at the ultrasound probe level. This allows the user to respect a suitable timing of the single deformation levels, such that at each

deformation level enough data can be collected. For example, a user interface may visually and/or acoustically and/or haptically indicate when to stop/change the deformation and/or whether an increasing or decreasing deformation is to be applied. Moreover, a feedback function may be used to indicate to the user, whether sufficient change of deformation between single steps is applied. For example, the method may be guided (i.e. a user be assisted) by the system to allow a reproducible predefined examination. In this context the deformation steps may have either predefined durations indicated by the user interface and/or their minimum duration may be determined as a function of the collected data amount in a deformation step. In the latter case, the system may decide based on the collected data (as for example described in context of fig. 4), whether enough valid data have been collected and a change of deformation may be applied.

It is also possible that the application of the deformation is automated, for example by using a robotic arm that moves the probe or further compression plate in an automated way according a predetermined/adaptative sequence.

Fig. 3a schematically shows a diagram of strain as a function of time frames according to embodiments of the present disclosure and fig. 3b schematically shows a diagram of elasticity E as a function of time frames corresponding to the strain function of fig. 3a. In other words, fig. 3a and 3b may be corresponding time diagrams, where fig. 3a shows a strain in % and fig. 3b an elasticity E. Both types of data are collected over time as respective data points (in other words as data samples). In the present schematic example, the data points of strain and elasticity are collected substantially at the same time. However, the data points may also be collected alternately, for example using a single ultrasound transducer or group of transducers.

In the example shown in fig. 3a and 3b, the level of deformation (i.e. the strain) changes in a stepwise manner over time. At each step, the level of deformation remains substantially constant (cf. fig. 3a). In each substantially constant level of deformation, approximately 5 to 8 data points are collected. However, also more or less data points may be collected.

As further shown in fig 3b, the elasticity data points collected during a change of deformation are rather noisy. For this reason, it is desirable that any SWE data point during a deformation change from one level to another is not collected/selected/sorted/used in the collection steps A1, A2, An, and/or not considered in the calculation step B1. In other words, during a change from one level of deformation to another only strain data may be collected to monitor the evolution of deformation.

Fig. 4 shows an exemplary flow chart of the method in more details, according to embodiments of the present disclosure. As explained in more details below, the flow chart of fig. 4 may comprise an acquisition sequence which has a plurality of different i.e. deformation (i.e. compression and/or decompression) levels indicated by a loop in the flow chart.

In a step S1 SWE and strain (i.e. deformation) data points are collected. Since step S1 may be carried out a plurality of times due to the loop from S3 over S4 to S1, it may correspond to steps A1, A2, A-n and A3 according to the present disclosure.

Strain may be estimated using Doppler phase between successive frames, which may be completed by using speckle tracking in B-mode image data. The B-mode speckle tracking allows to deal with large motion, and also by investigating speckle decorrelation one can detect out-of-plane motion. Under the latter case, the data points are desirably dropped.

In an optional step S2 it is determined whether the SWE and/or in particular the strain data points remain within a first predefined range. If they do not, the collected data may be disregarded and step S1 is repeated. At the same time, the user may be requested by the user interface to remain as far as possible at a constant level of compression.

If the SWE and/or in particular the strain data points remain within the first predefined range, the collected data are selected and/or recorded in step S3 for the present level of deformation in a data set 1...n. Accordingly, a data qualification condition may be applied in optional step S2 according to which only strain and/or Young's moduli of sufficient small variance may be retained.

The method then continues in step S4, in case the number of recorded data sets 1...n is less or equal to a predefined value N. N may be an integer, for example 2 or more. In step S4 a change of deformation (i.e. a compression or decompression) is applied to the medium. For example, step S4 may be triggered by the method when it is determined (for instance bayed on collected strain data points) that the level of deformation changes significantly (for example exceeding the predefined tolerance range). Desirably, only strain data points are collected during said deformation change. Step S4 may thus correspond to step A3 of the present disclosure. For example, a feedback (verbal, visual, ...) may be given to the user to move from one deformation level to, another once valid data of elasticity are collected and recorded in step S3.. The user may for example be guided by a visual indicator or text on display, or alternatively by an acoustic or haptic signal.

For example, step S4 may be carried out, until the level of deformation has reached a predefined threshold x. Said threshold X may be determined as a relative change of deformation with respect to the level of deformation in the prior collection step S1. For example, it might be defined as a predefined difference of strain, for example 5% increase or decrease of strain with respect to the level in previous step S1. Alternatively, step S4 may be carried out, until the change of deformation decreases and thus the level of deformation reaches a substantially constant level. For example, step S4 is carried out until the SWE and/or in particular the strain data points remain within the first predefined range or any other predefined range. In case in step S3, the number of data sets n (and hence the corresponding number of levels of deformation) become more than N, it may be determined that sufficient data have been collected. Then, the method continues with step S5. In step S5 the NL-SWE is estimated. Hence, step S5 may correspond to steps B1 and B2 of the present disclosure.

The NL-SWE model used in the present disclosure for estimating the NL-SWE may have a first-order model:

$$E(\epsilon) = E_0 - \frac{3A}{4}\epsilon \qquad (1)$$

where $E(\epsilon)$ is the Young's modulus under a strain constraint of strain $\epsilon$. $E_0 = E(0)$ is the constraint-free Young's modulus A is the NL-SWE parameter to estimate.

Summarized, in steps S1 to S4 the data are collected based on which the NL-SWE is estimated. In step S1 it is desired to calculate E and $\epsilon$ on different frames (i.e. at different times) when the probe motion is reduced (cf. step S2). The acquisition is guided to be carried out in desirably stationary stages, i.e. substantially constant levels of deformation. Accordingly, as long as the probe remains in a stage 0 (i.e. when the strain and desirably also the SWE is hence not varying too much) one or more data points are collected (cf. step S3). After acquisition at the current stage, the user may for example be requested to move uniaxially (press or release) to the next stage while the strain only is monitored (cf. S4). Said loop S1 to S4 may be repeated until enough data points are collected. The user may for example be guided by a visual indicator or text on display, or alternatively by an acoustic or haptic signal. Data may either be collected from for example a compression sequence or a depression sequence in the stages. Strain may be also be applied by pressing continuously or releasing continuously or by non-monotone steps following a target.

After the acquisition in steps S1 to S4, the data points may be processed in pair in step S5 to determine a respective gradient $R_{ij}$ by for example:

$$R_{ij} := \frac{E_i - E_j}{\epsilon_i - \epsilon_j} \qquad (2)$$

where E is a Young's modulus coefficient of the medium determined as a function of the wave elasticity data points and $\epsilon$ is a strain coefficient representing the level of deformation, wherein index i represents the first level and index j the second level of deformation.

The nonlinear elasticity coefficient A may be related to R by $A = -\frac{4}{3} \cdot$ R, such that the NL-SWE information can be quantified for each pair:

$$A_{ij} = -\frac{4}{3} \cdot \frac{E_i - E_j}{\epsilon_i - \epsilon_j} \qquad (3)$$

Given $N$ data points, the data quantity of these parameters are $(N - 1)N/2$. Hence, the data size to deal with can be increased from $N$ to $N(N-1)/2$ without increasing the acquisition duration.

In another example, the gradient $R_{i_1,...,i_k}$ may be generalized to

$$R_{i_1,i_2,...,i_k} := \frac{k\sum_{s=1,2,...,k}\epsilon_{i_s}E_{i_s} - \sum_{s=1,2,...,k}\epsilon_{i_s}\sum_{s=1,2,...,k}E_{i_s}}{k\sum_{s=1,2,...,k}\epsilon_{i_s}^2 - \left(\sum_{s=1,2,...,k}\epsilon_{i_s}\right)^2} \qquad (4)$$

where $i_1,i_2,...,i_k$ are indices representing $k$ $(k \geq 2)$ different levels of deformation. Accordingly, the slope defined on pairwise points (cf. for example eq. (2) above) may thus be generalized to slopes defined on a subset of points of size k (with k>2).

Then the variance of $A_{ij}$, i.e., $\sigma^2_{A_{ij}}$ may be computed. For example, this variance may be estimated by:

$$\sigma^2_{A_{ij}} := Var(A_{ij}) \approx \frac{16}{9} \cdot \left( \frac{\sigma^2_{E_{ij}}}{\mu^2_{\epsilon_{ij}}} + \frac{\mu^2_{E_{ij}} \sigma^2_{\epsilon_{ij}}}{\mu^4_{\epsilon_{ij}}} \right) \qquad (5)$$

where the mean $\mu_{E_{ij}}$ and variance $\sigma^2_{E_{ij}}$ of the difference of Young's moduli (i.e., $E_i - E_j$) may be estimated in the neighborhood of each pixel. Likewise, the mean $\mu_{\epsilon_{ij}}$ and variance $\sigma^2_{\epsilon_{ij}}$ of strain difference (i.e., $\epsilon_i - \epsilon_j$) may be estimated. The neighborhood may be spatial and/or temporal. For a temporal neighborhood, points within the same pair of the deformation-levels are desirably used to compute the mean and variance.

Optionally, a Geary-Hinkley Transformation (GHT) may be applied to improve the accuracy of the variance estimate, in particular with regard to a pixel correlation which can be better taken into account with the GHT:

$$\sigma^2_{A_{ij}} \approx \frac{16}{9} \cdot \frac{\sigma^2_{\epsilon_{ij}} R^2_{ij} - 2R_{ij} Cov_{E_{ij}, \epsilon_{ij}} + \sigma^2_{E_{ij}}}{\mu^2_{\epsilon_{ij}}} \qquad (6)$$

where $Cov_{E_{ij}, \epsilon_{ij}}$ denotes the covariance between $(E_i - E_j)$ and $(\epsilon_i - \epsilon_j)$. In other words, the GHT provides a more accurate estimation of the variance of $A_{ij}$. A minimum variance estimator, as described in the following, may use the variance of $A_{ij}$ as input to derive the optimal weights.

Then, a linear minimum-variance estimator may be formulated by optimizing

$$\arg \min_{w^*} Var\left( \sum_{ij} w_{ij} A_{ij} \right) \quad s.t. \, (subject\ to) \ \sum_{ij} w_{ij} = 1 \qquad (7)$$

, where $w_{ij}$ is a weighting score to be derived, and where $A_{ij}$ is a nonlinear shear wave elasticity parameter to be estimated.

The benefit of using the proposed linear minimum-variance estimator is that the estimation robustness and reproducibility may significantly increase.

The score $w_{ij}$ may be estimated by:

$$w^*_{ij} = \frac{\sigma^{-2}_{A_{ij}}}{\sum_{mn} \sigma^{-2}_{A_{mn}}} \qquad (8)$$

, where *Var* designates the variance, $w^*_{ij}$ is the score derived from the variances of each nonlinear shear wave elasticity parameter $A_{ij}$, $\sigma^{-2}_{A_{ij}}$ is an inverse variance, estimate of the nonlinear shear wave elasticity parameter $A_{ij}$ and $\sigma^{-2}_{A_{mn}}$ is an inverse variance estimate of the nonlinear shear wave elasticity parameter $A_{mn}$, wherein indexes m and n run through all pairwise combinations of shear wave elasticity data points by: 1<= m <= N, m < n <= N, where N is the number of data points.

The optimization produces the weights $w_{ij}$ that are applied on $A_{ij}$ to linearly weight each estimated parameter.

The final NL-SWE parameter estimate is given by this weighted average:

$$\hat{A} = \sum_{ij} w_{ij}^* A_{ij} \qquad\qquad (9)$$

Furthermore, the method may comprise after step S5, a confidence estimation step (not shown in fig. 4). Said step may comprise determining a statistical model on the confidence of the minimum-variance estimator as a function of the at least one score and the variance estimate $\sigma^2_{A_{ij}}$ of the nonlinear shear wave elasticity parameter $A_{mn}$, generating a confidence map comprising confidence information for the pixels of the nonlinear shear wave elasticity map. Accordingly, the variance of $\hat{A}$ may be computed by $\sum_{ij} w_{ij}^2 \sigma^2_{A_{ij}}$ to estimate confidence of the NL-SWE estimation.

Fig. 5 shows an exemplary embodiment of an ultrasound system according to embodiments of the present disclosure. The system 1 may be configured to estimate a nonlinear shear wave elasticity of a medium.

In particular, the ultrasound system 1 shown in figure 5 may be configured to provide ultrasound image data of a viscoelastic medium 2. In response to compressional ultrasound waves the system is scattering. The medium may be for example a human or animal body, for example a part of the body of a patient (breast, liver, abdomen, ...), in the case of clinical, or medical applications. This system 1 is also configured to monitor the propagation of elastic shear waves to allow providing image data of the elasticity of the medium 2.

The image data of the medium are produced, for example, by means of a processing unit (for example a computer or microcomputer) 4. The processing unit 4 may comprise for instance an input interface 4b such as a keyboard or the like, and an output interface 4a such as a screen or the like, located in same area or in different locations, including cloud computing parts) or any other electronic central unit, which causes compressional ultrasonic waves to be sent into the medium 2 from its outer surface 3. Said waves interact with scattering particles 5 contained in the medium 2, which particles are reflective for the compressional ultrasonic waves. The particles 5 can be constituted by any heterogeneity of the medium 2, and in particular, when it comes to a medical application, by collagen particles present in human tissues (these particles form on the ultrasound images points known as "speckle").

It is noted that the system shown in fig. 5 is a mere example. The system may also have any other architecture. For example, the part of the system may be distributed and/or remote to each other. In one example, the processing unit 4, the probe 6 and/or the electronic bay 7 may be located in different rooms or buildings.

To observe the medium 2 and to generate image data of the medium, an ultrasound probe 6 may be used, placed against the outer surface 3 of the observed medium 2. This probe sends, along a Z axis, compressional ultrasonic wave pulses of the type commonly used in echography, at a frequency of, for example, between 0.5 and 100 MHz and preferably between 0.5 and 15 MHz, for example of the order of 4 MHz.

The ultrasonic probe 6 may consist of an array of n ultrasonic transducers T1, T2, ..., Ti, ..., Tn, n being an integer greater than or at least equal to 1.

Probe 6 may be, for example, in the form of a linear array which can comprise, for example, n = 128 or 256 or 1024 transducers aligned along an X axis perpendicular to the Z axis. The probe in question may also be a two-dimensional or 3D array (planar or not) of transducers.

The transducers T1, T2, ... Tn may be controlled independently of each other by the processing unit 4, possibly by means of a central processing unit (CPU) which is contained, for example, in an electronic bay 7 connected by a flexible cable to the probe 6.

The transducers T1-Tn may thus selectively emit:

- either a "plane" compressional ultrasonic wave (i.e. in this case a wave whose wavefront is rectilinear in the X, Z plane) or any other type of unfocused wave illuminating the entire field of observation in the medium 2, for example a wave generated by having the various transducers T1-Tn emit random acoustic signals,
- or a compressional ultrasound wave focused on one or more points of the medium 2.

Optionally a synthetic imaging technique may be applied that uses several unfocused compressional waves, for example plane waves of different angles. Respective echo waves of these plane waves may be combined to obtain image data of the medium with improved quality.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

Although the present disclosure herein has been described with reference to particular embodiments, it is to be understood that these embodiments are merely illustrative of the principles and applications of the present disclosure.

It is intended that the specification and examples be considered as exemplary only, with a true scope of the disclosure being indicated by the following claims.

**Claims**

1. An ultrasonic method for estimating a nonlinear shear wave elasticity of a medium, the method comprising the following steps:

    A1. a first collection step in which a first set comprising one shear wave elasticity data point of the medium is collected at a first level of deformation applied to the medium,
    A2. a second collection step in which a second set comprising one shear wave elasticity data point of the medium is collected at a second level of deformation applied to the medium different to the first level,
    A3. a deformation estimation step in which the difference of deformation between the first and the second level of deformation is estimated,
    B1. a calculation step in which a gradient between at least two data points respectively belonging to the first and the second set is calculated as a function of the difference of deformation between the first and the second level of deformation,
    B2. an elasticity estimation step in which the nonlinear shear wave elasticity of the medium is estimated as a function of the gradient.

2. The method according to claim 1, wherein

    the gradient is determined by a variation of shear wave elasticity between the data points as a function of the difference of deformation between the first and the at least one second level of deformation, and/or
    the gradient $R_{ij}$ is determined by:

$$R_{ij} := \frac{E_i - E_j}{\epsilon_i - \epsilon_j}$$

    where E is a Young's modulus coefficient of the medium determined as a function of the wave elasticity data points and $\epsilon$ is a strain coefficient representing the level of deformation, wherein indices i and j represent two different levels of deformation, and/or
    the gradient $R_{i_1,i_2,...,i_k}$ is determined by:

$$R_{i_1,i_2,...,i_k} := \frac{k \sum_{s=1,2,...,k} \epsilon_{i_s} E_{i_s} - \sum_{s=1,2,...,k} \epsilon_{i_s} \sum_{s=1,2,...,k} E_{i_s}}{k \sum_{s=1,2,...,k} \epsilon_{i_s}^2 - \left(\sum_{s=1,2,...,k} \epsilon_{i_s}\right)^2}$$

    where indices $i_1$, $i_2$,..., $i_k$ represent k different levels of deformation.

3. The method according to claim 1 or 2, wherein
    the deformation estimation step comprises the sub-steps of:

    collecting a succession of strain data of the medium at the different levels of deformation,
    determining the difference of deformation between the first and the second level of deformation as a function of the strain data.

4. The method according to the preceding claim, wherein
    the sub-step of collecting a succession of strain data comprises:

generating a succession of ultrasound data of the medium, and
comparing the ultrasound data using a predefined deformation estimation algorithm.

5. The method according to claim 4, wherein
   the succession of strain data comprises three-dimensional strain data.

6. The method according to any one of the preceding claims, wherein
   a shear wave elasticity data point comprises a plurality of shearwave values referring respectively to different regions of a region of interest of the medium, wherein for each region a nonlinear shear wave elasticity parameter is estimated for constructing a nonlinear shear wave elasticity map of the region of interest.

7. The method according to any one of the preceding claims, wherein

   at least one of the first and second set comprises a plurality of shear wave elasticity data points,
   in the calculation step a plurality of gradients between at least two data points respectively belonging to the first and the second set is calculated as a function of the difference of deformation between the first and the second level of deformation, and
   in the elasticity estimation the nonlinear shear wave elasticity of the medium is estimated as a function of the plurality of gradients.

8. The method according to the preceding claims wherein
   the elasticity estimation step further comprises the sub-steps of:

   building a linear minimum-variance estimator based on the estimated gradients $R_{ij}$, $R_{i_1,i_2,...,i_k}$ and their statistical variances for estimating a score $w_{ij}$, $w_{i_1,i_2,...,i_k}$ for each gradient which is inversely proportional to its variance,
   weighting each gradient with the respective score,
   estimating a final NL-SWE parameter as a function of the weighted gradients and/or a weighted average of the gradients

9. The method according to any one of the preceding claims, wherein the linear minimum-variance estimator is determined by:

$$\arg\min_{w^*} Var\left(\sum_{ij} w_{ij}A_{ij}\right) \quad s.t.\,(subject\ to)\ \sum_{ij} w_{ij} = 1$$

, where

*Var* designates the variance,
$w_{ij}$ is an optimal weighting score to be derived,
$A_{ij}$ is a nonlinear shear wave elasticity parameter derived from the gradient $R_{ij}$, and indices i and j represent two different levels of deformation.

10. The method according to any one of the preceding claims, wherein
    a Geary-Hinkley Transformation is used for improving the accuracy of the minimum variance estimator.

11. The method according to any one of the preceding claims, wherein
    the optimal score $w_{ij}$ is estimated by:

$$w_{ij}^* = \frac{\sigma_{A_{ij}}^{-2}}{\sum_{mn} \sigma_{A_{mn}}^{-2}}$$

, where

$w_{ij}^*$ is the score derived from the variances of each nonlinear shear wave elasticity parameter $A_{ij}$,

$\sigma_{A_{ij}}^{-2}$ is an inverse variance estimate of the nonlinear shear wave elasticity parameter $A_{ij}$, and

$\sigma_{A_{mn}}^{-2}$ is an inverse variance estimate of the nonlinear shear wave elasticity parameter $A_{mn}$, wherein indices m and n run through at least two pairwise combinations of shear wave elasticity data points by: 1<= m <= N, m < n <= N, where N is the number of data points, and/or
the final NL-SWE parameter $\hat{A}$ is estimated by

$$\hat{A} = \sum_{ij} w_{ij}^* A_{ij} \, .$$

12. The method according to any one of the preceding claims, further comprising a confidence estimation step comprising:

   determining a statistical model on the confidence of the minimum-variance estimator $\hat{A}$ as a function of the at least one score and the variance estimate $\left(\sigma_{A_{ij}}^2\right)$ of the nonlinear shear wave elasticity parameter ($A_{ij}$),
   generating a confidence map comprising confidence information for the regions of the nonlinear shear wave elasticity map.

13. The method according to any one of the preceding claims, wherein
   during a transition from one level of deformation to another, only strain data are collected and/or any shear wave elasticity data point is not collected and/or considered in the calculation step.

14. The method according to any one of the preceding claims, wherein

   the same ultrasound probe is used to collect the shear wave elasticity data points and/or to collect the strain data and/or to apply the deformation to the medium, and/or
   the ultrasound probe used to collect the shear wave elasticity data points and/or to collect the strain data is configured for acquisition of two-dimensional or three-dimensional image data of the medium.

15. The method according to any one of the preceding claims, wherein
   further comprising a guidance and/or feedback step in which a user is guided by a user interface to apply the deformation to the medium and/or receives feedback from the user interface as a function of the applied deformation.

16. A computer program comprising computer-readable instructions which when executed by a data processing system cause the data processing system to carry out the method according to anyone of preceding method claims.

17. An ultrasonic system for estimating a nonlinear shear wave elasticity of a medium, the system being configured to:

   • collect a first set comprising one shear wave elasticity data point of the medium at a first level of deformation applied to the medium,
   • collect a second set comprising one shear wave elasticity data point of the medium at a second level of deformation applied to the medium different to the first level,
   • estimate the difference of deformation between the first and the second level of deformation,
   • calculate a gradient between the data points of at least two data points respectively belonging to the first and the second set as a function of the difference of deformation between the first and the second level of deformation,
   • estimate the nonlinear shear wave elasticity of the medium as a function of the gradient.

A1: Collection of a SWE data point at a first deformation level

↓

A2: Collection of a SWE data point at a second deformation level

↓

A-n (optional): Collection of a SWE data point at a nth deformation level

↓

A3: Estimation of the difference of deformation between the first and the second level of deformation

↓

B1: Calculation of a gradient between SWE data points as a function of the respective deformation change

↓

B2: Estimation of NL-SWE as a function of the gradient

**Fig. 1**

## FIG. 2A

## FIG. 2B

## FIG.2C

Fig. 3a

Strain change over frame

Fig. 3b

E change over frame

S1 (A1, A2, A-n, A3)

SWE+Strain

S4 (A3)

Strain only (until >x)

Var(SWE) and var(Strain)

>ε

<ε

S2

n+1

S3

compression

n>N

Calculation of A

S5 (B1, B2)

Fig. 4

**Fig. 5**

Europäisches
Patentamt

European
Patent Office

Office européen
des brevets

# EUROPEAN SEARCH REPORT

Application Number

EP 21 31 5133

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ARISTIZABAL SARA ET AL: "Application of Acoustoelasticity to Evaluate Nonlinear Modulus in Ex Vivo Kidneys", IEEE TRANSACTIONS ON ULTRASONICS, FERROELECTRICS, AND FREQUENCY CONTROL, IEEE, USA, vol. 65, no. 2, 1 February 2018 (2018-02-01), pages 188-200, XP011676451, ISSN: 0885-3010, DOI: 10.1109/TUFFC.2017.2781654 [retrieved on 2018-01-29] | 1-4,6,7, 12,16,17 | INV. A61B8/08 G01S7/52 |
| Y | * abstract; figures 2,3 * | 13-15 | |
| A | * page 190, left-hand column, paragraph 1 – paragraph 4 * * page 191, left-hand column, paragraph 2 – right-hand column, paragraph 1 * * page 192, left-hand column, paragraph 1 – page 195, right-hand column, paragraph 1; figures 4-7 * * page 196, right-hand column, paragraph 2 – page 198, left-hand column, paragraph 1 * * page 198, left-hand column, paragraph 3 * | 8-11 | |

-----

-/--

TECHNICAL FIELDS
SEARCHED (IPC)

A61B
G01S

The present search report has been drawn up for all claims

| Place of search | Date of completion of the search | Examiner |
|---|---|---|
| Munich | 18 January 2022 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 4**

Europäisches
Patentamt

European
Patent Office

Office européen
des brevets

**EUROPEAN SEARCH REPORT**

Application Number

EP 21 31 5133

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GOSWAMI SOUMYA ET AL: "Nonlinear Shear Modulus Estimation With Bi-Axial Motion Registered Local Strain", IEEE TRANSACTIONS ON ULTRASONICS, FERROELECTRICS, AND FREQUENCY CONTROL, IEEE, USA, vol. 66, no. 8, 1 August 2019 (2019-08-01), pages 1292-1303, XP011737055, ISSN: 0885-3010, DOI: 10.1109/TUFFC.2019.2919600 [retrieved on 2019-07-30] * abstract * * page 1293, left-hand column, paragraph 2 - page 1294, left-hand column, paragraph 1; figures 2,3 * * page 1295, left-hand column, paragraph 1 - page 1296, left-hand column, paragraph 1 * | 1,3-5, 16,17 | |
| X | ARISTIZABAL SARA ET AL: "Application of acoustoelasticity to evaluate non-linear modulus in ex vivo kidneys", 2016 IEEE INTERNATIONAL ULTRASONICS SYMPOSIUM (IUS), IEEE, 18 September 2016 (2016-09-18), pages 1-4, XP032988696, DOI: 10.1109/ULTSYM.2016.7728814 [retrieved on 2016-11-01] * abstract * * page 1, right-hand column, paragraph 5 - page 2, left-hand column, paragraph 1; figures 1,2 * | 1,2,6, 16,17 | TECHNICAL FIELDS SEARCHED (IPC) |

-/--

The present search report has been drawn up for all claims

| Place of search | Date of completion of the search | Examiner |
|---|---|---|
| Munich | 18 January 2022 | Daoukou, Eleni |

EPO FORM 1503 03.82 (P04C01)

## EUROPEAN SEARCH REPORT

Application Number

EP 21 31 5133

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | LATORRE-OSSA H ET AL: "Quantitative imaging of nonlinear shear modulus by combining static elastography and shear wave elastography", IEEE TRANSACTIONS ON ULTRASONICS, FERROELECTRICS, AND FREQUENCY CONTROL, IEEE, USA, vol. 59, no. 4, 1 April 2012 (2012-04-01), pages 833-839, XP011491387, ISSN: 0885-3010, DOI: 10.1109/TUFFC.2012.2262 * page 834, left-hand column, paragraph 3 - page right, column 1 * * page 837, left-hand column, paragraph 1 * | 1,16,17 | |
| X,D | BERNAL MIGUEL ET AL: "In Vivo Quantification of the Nonlinear Shear Modulus in Breast Lesions: Feasibility Study", IEEE TRANSACTIONS ON ULTRASONICS, FERROELECTRICS, AND FREQUENCY CONTROL, IEEE, USA, vol. 63, no. 1, 1 January 2016 (2016-01-01), pages 101-109, XP011596535, ISSN: 0885-3010, DOI: 10.1109/TUFFC.2015.2503601 [retrieved on 2015-12-30] * page 102, paragraph 3 - paragraph 5; figure 3 * * page 103, left-hand column, paragraph 2 - paragraph 4 * * page 103, right-hand column, paragraph 2 - page 104, left-hand column, paragraph 1 * | 1,16,17 | TECHNICAL FIELDS SEARCHED (IPC) |

-/--

The present search report has been drawn up for all claims

| Place of search | Date of completion of the search | Examiner |
|---|---|---|
| Munich | 18 January 2022 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 21 31 5133

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2021/116326 A2 (SUPERSONIC IMAGINE [FR]) 17 June 2021 (2021-06-17) | 13-15 | |
| A | * page 1, line 5 – line 10 *<br>* page 2, line 15 – line 27 *<br>* page 3, line 5 – page 9, line 21 *<br>----- | 1,12,16,17 | |
| A | GOSWAMI SOUMYA ET AL: "Quantitative nonlinear shear modulus mapping using freehand scanning",<br>2020 IEEE INTERNATIONAL ULTRASONICS SYMPOSIUM (IUS), IEEE,<br>7 September 2020 (2020-09-07), pages 1-3,<br>XP033859224,<br>DOI: 10.1109/IUS46767.2020.9251339<br>[retrieved on 2020-11-06]<br>* page 2, left-hand column, paragraph 1 – right-hand column, paragraph 1; figure 1 *<br>----- | 1,16,17 | |

**TECHNICAL FIELDS SEARCHED (IPC)**

The present search report has been drawn up for all claims

| Place of search | Date of completion of the search | Examiner |
|---|---|---|
| Munich | 18 January 2022 | Daoukou, Eleni |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 21 31 5133

This annex lists the patent family members relating to the patent documents cited in the above-mentioned European search report.
The members are as contained in the European Patent Office EDP file on
The European Patent Office is in no way liable for these particulars which are merely given for the purpose of information.

18-01-2022

| Patent document cited in search report | | Publication date | Patent family member(s) | | Publication date |
|---|---|---|---|---|---|
| WO 2021116326 | A2 | 17-06-2021 | FR | 3104736 A1 | 18-06-2021 |
| | | | WO | 2021116326 A2 | 17-06-2021 |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021116326 A2

**Non-patent literature cited in the description**

- **H. LATORRE-OSSA.** Quantitative imaging of nonlinear shear modulus by combining static elastography and shear wave elastography. *IEEE Transactions on Ultrasonic Ferroelectronics and Frequency Control,* April 2012, vol. 59 (4), 833-9

- **M. BERNAL.** In Vivo Quantification of the Nonlinear Shear Modulus in Breast Lesions: Feasibility Study. *IEEE Trans Ultrasonic Ferroelectric Frequency Control,* 24 November 2015, vol. 63 (1), 101-9